# FASCICULE DE BREVET EUROPEEN

(11) **EP 1 002 514 B1**
(45) Date de publication et mention de la délivrance du brevet: **12.06.2002**
(21) Numéro de dépôt: 99402508.8
(22) Date de dépôt: 12.10.1999
(51) Int. Cl.: A61K 7/00, A61K 7/043, A61K 7/021, A61K 7/48, A61K 7/42

(54) **Composition cosmétique à phase continue lipophile contenant un pigment vanadate de bismuth**
Kosmetische Zusammensetzung mit einer kontinuierlichen lipophilen Phase enthaltend ein Bismuth Vanadat Pigment
Cosmetic composition with a continuous lipophilic phase containing a bismuth vanadate pigment

(30) Priorité: 10.11.1998 FR 9814160
(43) Date de publication de la demande: 24.05.2000
(73) Titulaire: L'OREAL, 75008 Paris (FR)
(72) Inventeur: Lemann, Patricia, 94000 Créteil (FR)
(74) Mandataire: Lhoste, Catherine

(56) Documents cités:
- EP-A- 0 551 637
- EP-A- 0 632 110
- DE-A- 3 315 850

## Description

La présente invention se rapporte à des compositions cosmétiques à phase continue lipophile contenant un nouveau pigment jaune de couleur intense et saturée, non générateur de radicaux libres, et plus spécialement à des compositions de maquillage de la peau aussi bien du visage que du corps humain, des lèvres et des phanères comme les ongles, les cils, les sourcils ou les cheveux.

Les compositions de maquillage telles que les poudres libres ou compactées, les fonds de teint, les fards à joues ou à paupières, les rouges à lèvres, les anti-cernes, les blushes, les mascaras, les eye-liners, les crayons à lèvres ou à yeux ou encore les produits de maquillage du corps, sont constituées d'un véhicule approprié et d'agents de coloration de natures différentes, destinés à conférer une certaine couleur à ces compositions, avant et/ou après leur application sur la peau, les lèvres et/ou les phanères.

Ces agents de coloration peuvent être des laques, des pigments minéraux ou organiques et/ou des pigments nacrés ou encore des colorants. Dans la gamme des pigments jaunes, les cosméticiens disposent de pigments d'origine minérale comme les oxydes de fer jaunes et de pigments d'origine organique. Les pigments minéraux, et en particulier les oxydes minéraux ont l'avantage d'être relativement stables mais ont l'inconvénient de donner des couleurs plutôt ternes et pâles. Les laques organiques ont l'avantage de conférer aux compositions des couleurs vives, mais sont pour la plupart instables à la lumière, à la température ou au pH. Certaines de ces laques présentent également l'inconvénient de tacher la peau ou les ongles de manière disgracieuse après application, par dégorgement du colorant. Les pigments nacrés quant'à eux permettent d'obtenir des couleurs variées, mais jamais intenses, à effets irisés mais le plus souvent assez faibles.

Par ailleurs, certains agents de coloration présentent l'inconvénient d'engendrer des radicaux libres dans les formules de maquillage modifiant le rendu des couleurs et la stabilité des compositions, puis sur la peau après application, ce qui favorise le vieillissement cutané (apparition de rides, ridules, jaunissement de la peau). En particulier, les oxydes de fer jaune provoquent souvent une oxydation des huiles polyinsaturées (huiles végétales par exemple), ce qui limite la gamme de compositions. Comme agents de coloration présentant cet inconvénient ont peut citer notamment les mélanges d'oxydes de fer jaune (Cl:77492) vendu sous la dénomination commerciale « Sicovit Jaune 10 E 172 » par BASF, les pigments d'origine organique ; la laque d'aluminium de tartrazine sur alumine (20/80) (Cl :19140, Cl: 77002) vendu sous le nom commercial FD & C Yellow 5 de Warner Jenkinson.

Aujourd'hui pour pallier cet inconvénient, on utilise des agents anti-oxydants comme par exemple l'éthoxyquine. Malheureusement, il est souvent difficile de trouver un agent anti-oxydant efficace à 100% compte tenu de la multiplicité des ingrédients présents dans les compositions de maquillage. De plus, les agents anti-oxydants engendrent souvent eux-mêmes des produits de dégradation (oxydation de l'agent anti-oxydant) qui peuvent être gênants.

La présente invention a justement pour objet l'utilisation dans une composition à application topique d'un nouveau pigment jaune de couleur intense et saturée, stable ayant l'avantage d'engendrer beaucoup moins de radicaux libres que les pigments classiquement utilisés notamment pour obtenir une couleur jaune.

De façon surprenante, le demandeur a trouvé que le vanadate de bismuth de formule BiVO₄ permettait de limiter la production de radicaux libres, puisqu'il a la propriété d'engendrer très peu de radicaux libres, et de limiter ainsi l'utilisation d'anti-oxydants dans les compositions. En outre ce pigment permet d'obtenir une coloration intense et en particulier un jaune citron, très vif et intense, de très grande pureté de couleur, non dégorgeante sur les matières kératiniques, stable à la lumière, au pH et à la température. Il permet, en outre, d'élargir la gamme de couleur dans le domaine cosmétique et de participer à la création de verts éclatants en mélange avec des bleus, d'oranges vifs. De plus, il permet de réduire la quantité de pigments de l'art antérieur, permettant ainsi de préserver la brillance de la composition ainsi que celle du film déposé, ce qui est très recherché pour les produits de maquillage des lèvres.

De façon plus précise, l'invention a pour objet une composition à application topique colorée à phase continue lipophile et plus spécialement une composition cosmétique de maquillage à phase continue lipophile contenant du vanadate de bismuth (BiVO₄) comme agent de coloration et plus précisément de pigment.

La fabrication de ce pigment est en particulier celle décrite dans les documents EP-A-551637, EP-A-632110 de BASF. Il présente en particulier une taille de 0,3 µm environ.

Le vanadate de bismuth peut cristalliser sous différentes formes et exprimer, ainsi des tons de jaunes différents :
- réseau monoclinique ; la fergusonite : jaune brillant, densité 6,959
- réseau tétragonal ; zircon : jaune pâle, densité 6,127
- réseau tétragonal ; scheelite : jaune brillant, densité 6,929
- réseau orthorhombique ; pulchérite : jaune pâle.

Il peut se présenter aussi sous forme pure ou déposé sur un substrat. En particulier il se présente sous forme pure et est commercialisé sous la référence Sicopal Gelb L1100 par la société BASF.

Pour montrer la propriété qu'a le vanadate de bismuth de ne pas engendrer de radicaux libres, le demandeur a réalisé un test d'éthylène selon le procédé décrit dans l'article « Ethylene formation from methionine as a method to evaluate oxygen free radical scavenging and metal inactivation by cosmetics » de J.-B. Galey, F. Millecamps et Q.-L. Nguyen, *International Journal of Cosmetic Science,* 13, 65-78, 1991.

L'objectif est de comparer le comportement du pigment minéral selon l'invention, par rapport à celui de pigments classiques dans un test de photo-oxydation utilisant le fer comme générateur de radicaux libres.

Dans le protocole de mesure du test éthylène, le FeCl₃ utilisé pour activer la production de radicaux libres a été substitué par chacun des pigments à tester. Les résultats sont donnés dans le tableau ci-après.

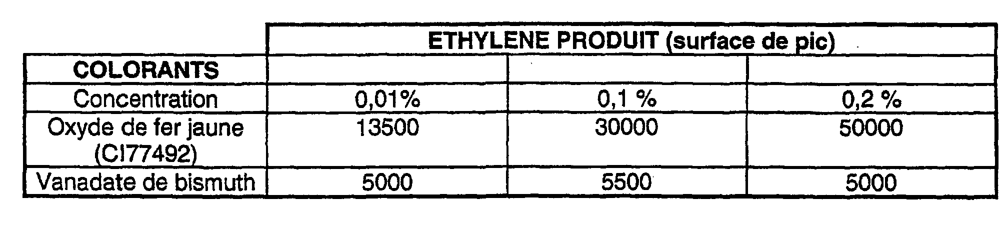

Le témoin FeCl3 à 0,005 % étant en moyenne à 9000 (unité arbitraire - mesure relative).

Plus la quantité d'éthylène est élevée, plus la production de radicaux libres est élevée.

L'oxyde de fer jaune (Cl :77492) n'est pas inerte. A faible dose, il active jusqu'à une certaine concentration où l'effet protecteur du pigment commence à jouer, tandis que pour le vanadate de bismuth, le taux d'éthylène produit est très faible et n'évolue pas en fonction de la concentration. Ce pigment pourra donc être avantageusement utilisé dans des compositions de maquillage et des compositions colorées solaires notamment destinées à la protection de la peau et/des muqueuses telles que les lèvres, sans engendrer de radicaux libres et donc limiter ainsi la dégradation de la peau et/ou des lèvres.

Le pigment de la composition de l'invention présente, en outre, par rapport aux oxydes de fer jaunes couramment utilisés en cosmétique, l'avantage d'être plus saturé en couleur, d'être plus vif, de couleur plus intense, ce qui permet, notamment de l'utiliser en plus faible quantité, pour un rendu de couleur équivalent. On donne ci-après les paramètres colorimétriques du vanadate de bismuth vendu par la société BASF sous la marque commerciale Sicopal Gelb L 1100 par rapport à celui de l'oxyde de fer jaune (Cl :77492).

| Paramètres | Oxyde de fer jaune | vanadate de bismuth |
|---|---|---|
| L | 69,9 | 92,8 |
| a | 6,85 | -12,92 |
| b | 50,35 | 84,64 |
| c | 50,6 | 85,27 |

Plus la valeur de c est élevée, plus la couleur est saturée : le vanadate de bismuth a une couleur plus vive, plus intense que l'oxyde de fer, ce qui permet de colorer fortement une composition cosmétique avec une faible quantité de pigment. Ainsi les problèmes de rhéologie (difficulté d'application, maquillage hétérogène) liés à une trop forte quantité de pigments dans les compositions de l'art antérieur sont largement atténués.

Par rapport à une laque organique, le vanadate de bismuth est plus couvrant, à quantité de pigment égale.

Le pigment selon de l'invention peut être incorporé dans une composition cosmétique ou dermatologique à phase continue lipophile notamment de maquillage en une quantité déterminable aisément par l'homme du métier sur la base de ses connaissances générales, et qui peut notamment aller de 0,01 à 50% en poids par rapport au poids de la composition, de préférence en une quantité allant de 0,5 à 25% en poids. Même à concentration élevée, ce pigment ne déstructure pas la composition.

La composition de l'invention peut se présenter sous forme d'un produit à appliquer sur les lèvres, les yeux, la peau et/ou les phanères d'êtres humains. Elle contient donc un milieu cosmétiquement acceptable, c'est-à-dire un milieu compatible avec toutes les matières kératiniques telles que la peau aussi bien du corps humain que du visage, les ongles, les cheveux, les cils et sourcils.

Selon l'invention, ce milieu contient une phase continue lipophile, c'est-à-dire un mélange d'un ou plusieurs corps gras ou de solvants organiques, non miscibles à l'eau, qui peuvent être liquides, pâteux ou solides à température ambiante (25°C en général). En particulier, ce milieu peut comprendre ou se présenter notamment sous forme de suspension, dispersion ou solution dans une phase huileuse ou de solvant organique lipophile, éventuellement épaissie, voire gélifiée ; suspension ou dispersion dans une phase cireuse ; émulsion eau-dans-huile (E/H), ou multiple (H/E/H) sous forme de crème de pâte ou même de solide ; gel anhydre ou mousse huileux ; gel émulsionné ; lotion biphase ou multiphase ; spray ; poudre libre, compactée ou coulée ; pâte anhydre. L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

La composition comprend donc une phase continue lipophile qui peut contenir des corps gras liquides à température ambiante et pression atmosphérique, appelés souvent huiles, des solvants organiques non miscibles à l'eau, des cires, des gommes, des corps gras pâteux ou un mélange de ces constituants. Cette phase continue peut représenter de 0,5 à 99,99% du poids total de la composition.

Lorsque la composition selon l'invention se présente sous la forme d'une émulsion, elle peut éventuellement comprendre, en outre, un tensioactif, de préférence en une quantité de 0 à 30% et notamment de 0,01 à 30% en poids par rapport au poids total de la composition.

Selon l'application envisagée, la composition peut comprendre, en outre, un polymère filmogène (comme les polyuréthannes, les polyacryliques, les hybrides polyuréthannes et polyacryliques, les polyesters, la nitrocellulose, les résines hydrocarbonées et/ou siliconées). Ceci est notamment le cas lorsqu'on souhaite préparer une composition du type vernis à ongle, mascara, eye-liner, laque à lèvres ou une composition capillaire du type laque. Les polymères peuvent être dissous ou dispersés dans le milieu cosmétiquement acceptable et éventuellement associés à des agents de coalescente et/ou des plastifiants.

Comme huiles, utilisables dans l'invention, on peut citer : les huiles hydrocarbonées d'origine animale telles que le perhydrosqualène ; les huiles hydrocarbonées végétales telles que les triglycérides liquides d'acides gras de 4 à 10 atomes de carbone comme les triglycérides des acides heptanoïque ou octanoïque, ou encore les huiles de tournesol, de maïs, de soja, de pépins de raisin, de sésame, d'abricot, de macadamia, de ricin, d'avocat, les triglycérides des acides caprylique/caprique, l'huile de jojoba, de beurre de karité ; les hydrocarbures linéaires ou ramifiés, d'origine minérale ou synthétique tels que les huiles-de paraffine et leurs dérivés, la vaseline, les polydécènes, le polyisobutène hydrogéné tel que le parléam ; les esters et les éthers de synthèse notamment d'acides gras ou d'alcool gras ayant de 8 à 26 atomes de carbone, comme par exemple l'huile de Purcellin, le myristate d'isopropyle, palmitate d'éthyl-2hexyle, stéarate d'octyl-2-dodécyle, érucate d'octyl-2-dodécyle, l'isostéarate d'isostéaryle ; les esters d'acides gras ou d'alcool gras hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras ; des esters de polyol comme le dioctanoate de propylène glycol, le diheptanoate de néopentylglycol, le diisononanoate de diéthylèneglycol ; et les esters du pentaérythritol ; des alcools gras ayant de 12 à 26 atomes de carbone comme l'octyldodécanol, le 2-butyloctanol, le 2-hexyldécanol, le 2-undécylpentadécanol, l'alcool oléique ; les huiles fluorées partiellement hydrocarbonées et/ou siliconées ; les huiles siliconées comme les polyméthylsiloxanes (PDMS) volatiles ou non, linéaires ou cycliques, liquides ou pâteux à température ambiante comme les cyclométhicones, les diméthicones, comportant éventuellement un groupement phényle, comme les phényl triméthicones, les phényltriméthylsiloxydiphényl siloxanes, les diphénylméthyldiméthyl-trisiloxanes, les diphényl diméthicones, les phényl diméthicones, les polyméthylphényl siloxanes ; leurs mélanges.

Ces huiles peuvent représenter de 0 à 99,99 % en poids par rapport au poids total de la phase grasse.

La phase continue lipophile de la composition selon l'invention peut également comprendre un ou plusieurs solvants organiques, cosmétiquement acceptables (tolérance, toxicologie et toucher acceptables). Ces solvants organiques peuvent représenter de 0 à 90 % du poids total de la composition et peuvent être choisis dans le groupe constitué par les solvants organiques lipophiles, les solvants amphiphiles ou leurs mélanges. Comme solvants organiques utilisables dans la composition de l'invention, on peut citer les esters de l'acide acétique comme l'acétate de méthyle, d'éthyle, de butyle, d'amyle, de méthoxy-2-éthyle ; les cétones comme la méthyléthylcétone, la méthylisobutylcétone, l'acétate d'isopropyle ; les hydrocarbures comme le toluène, le xylène, le p-cylène, l'hexane , l'heptane ; les aldéhydes ayant de 5 à 10 atomes de carbone ; les éthers ayant au moins 3 atomes de carbones et leurs mélanges.

La composition de l'invention peut, en outre, comprendre une phase particulaire additionnelle pouvant être présente à raison de 0 à 30 % du poids total de la composition, de préférence de 0,05 à 20 % et qui peut comprendre des pigments et/ou des nacres et/ou des charges utilisés dans les compositions cosmétiques.

Par pigments, il faut comprendre des particules blanches ou colorées, minérales ou organiques, insolubles dans la phase grasse liquide, destinées à colorer et/ou opacifier la composition. Par charges, il faut comprendre des particules incolores ou blanches, minérales ou de synthèse, lamellaires ou non lamellaires. Par nacres, il faut comprendre des particules irisées, notamment produites par certains mollusques dans leur coquille ou bien synthétisées. Ces charges et nacres servent notamment à modifier la texture de la composition.

Les pigments autres que le vanadate de bismuth peuvent être présents dans la composition à raison de 0 à 25 % du poids de la composition finale, et de préférence à raison de 2 à 15 %. Comme pigments minéraux utilisables dans l'invention, on peut citer les oxydes de titane, de zirconium ou de cérium ainsi que les oxydes de zinc, de fer ou de chrome et le bleu ferrique. Parmi les pigments organiques utilisables dans l'invention, on peut citer le noir de carbone, et les laques de baryum, strontium, calcium, aluminium ou encore les dicéto pyrrolopyrrole (DPP) décrits dans les documents EP-A-542669, EP-A-787730, EP-A-787731 et WO 96/08537.

Les nacres peuvent être présentes dans la composition à raison de 0 à 20 % du poids total de la composition, de préférence à un taux de l'ordre de 1 à 15 %. Parmi les nacres utilisables dans l'invention, on peut citer le mica recouvert d'oxyde de titane, d'oxyde de fer, de pigment naturel ou d'oxychlorure de bismuth tel que le mica titane coloré.

Les charges peuvent être présentes à raison de 0 à 30 % du poids total de la composition, de préférence 0,5 à 15 %. On peut notamment citer le talc, le stéarate de zinc, le mica, le kaolin, les poudres de Nylon (Orgasol notamment) et de polyéthylène, le Téflon, l'amidon, le nitrure de bore, des microsphères de copolymères telles que l'Expancel (Nobel Industrie), le polytrap (Dow Corning) et les microbilles de résine de silicone (Tospearl de Toshiba, par exemple).

La composition de l'invention peut comprendre, avantageusement une phase grasse solide ou pâteuse, contenant une ou plusieurs gommes et/ ou une ou plusieurs cires et/ou un ou plusieurs corps gras pâteux. Les cires et les corps gras pâteux peuvent être hydrocarbonés, fluorés et/ou siliconés et être d'origine végétale, minérale, animale et/ou synthétique. En particulier, les cires présentent une température de fusion supérieure à 45 °C et les corps gras pâteux des point de fusion de 25 °C à 45 °C.

Comme cire utilisable dans la composition de l'invention, on peut citer la cire d'abeilles, la cire de Carnauba ou de Candellila, la paraffine, les cires microcristallines, la cérésine ou l'ozokérite ; les cires synthétiques comme les cires de polyéthylène ou de Fischer Tropsch, les cires de silicones comme les alkyl ou alkoxy-diméticone ayant de 16 à 45 atomes de carbone.

Les gommes sont généralement des PDMS à haut poids moléculaire et les corps pâteux sont généralement des composés hydrocarbonés comme les lanolines et leurs dérivés ou encore des PDMS.

La nature et la quantité des corps solides sont fonction des propriétés mécaniques et des textures recherchées. A titre indicatif, la composition peut contenir de 0 à 50 % en poids de cires, par rapport au poids total de la composition et mieux de 1 à 30 %.

La composition selon l'invention peut, de plus, comprendre tous les ingrédients classiquement utilisés dans les domaines concernés et plus spécialement dans les domaines cosmétique et dermatologique. Ces ingrédients sont en particulier choisis parmi les conservateurs, les épaississants de phase aqueuse (biopolymères polysaccharidiques, les polymères synthétiques) ou grasse, les parfums, les actifs hydrophiles ou lipophiles et leurs mélanges. Les quantités de ces différents ingrédients sont celles classiquement utilisées dans les domaines concernés et par exemple de 0 à 20 % et notamment de 0,01 à 15 % du poids total de la composition. La nature de ces ingrédients et leur proportion doit être compatibles avec l'obtention de compositions selon l'invention, stables épaissies et brillantes.

La composition peut contenir une phase aqueuse, alcoolique ou hydroalcoolique, sous forme dispersée ou émulsionnée dans la phase continue. Cette phase peut contenir de l'eau, des alcools, un mélange d'eau et d'alcool ou d'acétone. Les alcools sont notamment des monoalcools inférieurs linéaires ou ramifiés ayant de 2 à 5 atomes de carbone comme l'éthanol ou le propanol, des polyols comme la glycérine, la diglycérine, le propylène glycol, le sorbitol, le penthénol, le penthylène glycol, les polyéthylène glycols. Cette phase aqueuse peut représenter de 0 à 70% du poids de la composition. Elle peut, en outre, contenir des éthers en C₂ et des aldéhydes en C₂-C₄.

La composition selon l'invention peut avoir l'aspect d'une crème, pommade, lotion fluide, de pâte souple de viscosité dynamique à 25°C de l'ordre de 1 à 40 Pa.s, onguent, solide coulé ou moulé et notamment en stick ou en coupelle.

Cette composition peut être avantageusement utilisée pour le maquillage de la peau et/ou des lèvres et/ou des phanères selon la nature des constituants utilisés. En particulier, la composition de l'invention peut être un bâton de rouge à lèvres ou une laque à lèvres, un brillant à lèvres (gloss en terminologie anglo-saxonne) utilisable tel quel ou pour appliquer sur un film de rouge à lèvres notamment pour en augmenter sa brillance et/ou sa couleur (top coat en terminologie anglo-saxonne). Elle peut aussi constituer un fond de teint solide, un produit anti-cernes ou contours des yeux, un eye liner, un mascara, une ombre à paupières, une poudre compactée, un blush, un vernis à ongle. Ces compositions peuvent, en outre, contenir des actifs cosmétiques ou dermatologiques, en vue, notamment d'apporter un aspect soin ou traitant à la composition. Ainsi la composition peut contenir des vitamines et autres actifs lipophiles (lanoline, filtre UVA) ou hydrophiles (hydratants comme la glycérine).

L'invention a encore pour objet une utilisation cosmétique de la composition ci-dessus pour soigner et/ou maquiller et/ou protéger la peau et/ou les lèvres et/ou les phanères d'êtres humains ainsi qu'une utilisation de cette composition pour la préparation d'un onguent destiné à traiter et/ou protéger la peau et/ou les lèvres et/ou les phanères. L'invention a aussi pour objet un procédé de traitement cosmétique de la peau et/ou des lèvres et/ou des phanères, consistant à appliquer sur la peau et/ou les lèvres et/ou les phanères d'êtres humains la composition définie ci-dessus.

De façon plus spécifique, l'invention a pour objet un produit à lèvres, un fond de teint ou un vernis à ongle.

La composition de l'invention peut être obtenue selon les procédés de préparation classiquement utilisés en cosmétique ou en dermatologie.

L'invention a encore pour objet l'utilisation, pour la fabrication d'une composition cosmétique ou d'une composition dermatologique, colorée, d'un agent de coloration tel que décrit ci-dessus, afin de protéger la peau et/ou les lèvres et/ou les phanères contre les méfaits des radicaux libres et/ou lutter contre les signes cutanés du vieillissement notamment photoinduit. Ces signes du vieillissement sont en particulier les rides, ridules, la peau flasque et/ou jaunie.

L'invention a encore pour objet un procédé non thérapeutique de protection de la peau et/ou des lèvres et/ou des phanères, contre les signes cutanés du vieillissement photoinduit, consistant à appliquer sur la peau et/ou les lèvres et/ou les phanères la composition telle que définie ci-dessus.

Les exemples de compositions ci-après sont donnés à titre illustratif. Les pourcentages sont des pourcentages en poids.

### Exemple 1 : de fond de teint de type E/H

### - Composition

. Cyclopentasiloxane 25,05 %
. Diméthicone copolyol dans cyclopentasiloxane * 6,00 %
. Isododécane 4,55 %
. Pigments
   Oxyde de fer rouge-brun 0.67 %
   Oxyde de fer noir 0.25%
   Dioxyde de titane 7.63 %
   Vanadate de bismuth (Sicopal Gelb L1100) 0.75 %
. Poudre de nylon 8,00 %
. Parfum 0,60 %
. Silicate de magnésium 0,60 %
. Gomme de cellulose 3,50 %
. Succinate d'isostéaryle de diglycéryle 2,00 %
. Eau qsp 100 %

### - Mode opératoire :

On prédisperse les pigments dans le mélange de cyclométhicones puis on homogénéise la phase grasse (tensioactifs + huiles) à40°-50° C. On laisse refroidir puis on ajoute les pigments prédispersés. On incorpore sous agitation dans la phase grasse l'ensemble de la phase aqueuse préalablement homogénéisée. On maintient une agitation moyenne jusqu'à émulsification.

On obtient un fond de teint de couleur beige, de couvrance élevée, stable à la lumière, ne laissant aucune griffe (ou marque) après démaquillage.

### Exemple 2 : rouge à lèvres anhydre

### - Composition

. Esters d'alcools gras en C₈-C₁₀ 30,0 %
. Alcool gras en C₁₀ 7,0 %
. Amidon octylsuccinate d'aluminium 10,0 %
. Ozokérite 10,0 %
. Cire de carnauba 4,0 %
. Cire d'abeille 3,0 %
. Dioxyde de titane 3,0%
. Vanadate de bismuth (Sicopal Gelb L1100) 1,0 %
. Rouge Flaming (Cl12085) 1,0 %
. Parfum 0,5 %
. Huile de riçin qsp 100 %

### - Mode opératoire :

Les pigments sont broyés dans le mélange d'alcools gras, d'esters d'alcools gras et d'huile de ricin. L'ozokérite, la cire de Carnauba et la cire d'abeille sont fondues extemporanément dans un récipient et sont introduites dans le mélange sous agitation continue. On introduit sous vide l'amidon octylsuccinate d'aluminium et enfin on incorpore le parfum. On coule l'ensemble dans un moule approprié pour obtenir un stick puis on laisse refroidir le tout jusqu'à la température ambiante.

On obtient un rouge à lèvres de couleur orange, stable et couvrant.

### Exemple 3 : vernis à ongles anhydre

### - Composition

. Nitrocellulose 10,9 %
. Résine toluène sulfonamide formaldéhyde (Ketjenflex MS80 vendu par AKZO) 10,7 %
. Acétyltributyle citrate (Citroflex A4 vendu par PFIZER) 6,5 %
. Toluène 31,0 %
. Acétate de butyle 21,6 %
. Acétate d'éthyle 9,3 %
. Alcool isopropylique 7,7 %
. Stéaralkonium hectorite 1,3 %
. Vanadate de bismuth (Sicopal Gelb L1100) 1,0 %
. Acide citrique 0,1 %

### - Mode opératoire :

On mélange à température ambiante tous les constituants de la composition.

On obtient un vernis à ongles d'un jaune éclatant, brillant, stable à la lumière.

## Revendications

1. Composition à application topique colorée à phase continue lipophile, **caractérisée par le fait qu'**elle comprend, du vanadate de bismuth comme agent de coloration.

2. Composition selon la revendication 1, **caractérisée en ce que** l'agent de coloration est présent à raison de 0,01 à 50% en poids par rapport au poids de la composition .

3. Composition selon la revendication 1 ou 2, **caractérisée en ce que** l'agent de coloration est présent à raison de 0,5 à 25% en poids par rapport au poids de la composition.

4. Composition selon l'une des revendications précédentes, se présentant sous forme d'un produit de maquillage de la peau, des lèvres et/ou des phanères d'être humain.

5. Composition selon l'une des revendications précédentes, se présentant sous forme de mascara, eye-liner ou composition capillaire de type laque, produit à lèvres, brillant à lèvres, fond de teint, produit anti-cernes, fard à joues ou à paupières, maquillage du corps, poudres ou vernis à ongles.

6. Composition selon l'une des revendications précédentes, **caractérisée en ce que** la phase continue grasse contient au moins un corps gras choisi parmi les huiles, les cires, les gommes, les corps gras pâteux, les solvants organiques lipophiles et leurs mélanges.

7. Composition selon l'une des revendications précédentes, comprenant, en outre, au moins une phase aqueuse, alcoolique ou hydroalcoolique dispersée ou émulsionnée dans la phase lipophile.

8. Composition selon l'une des revendications précédentes, **caractérisée en ce qu'**elle contient, en outre, une phase particulaire additionnelle pouvant être présente à raison de 0 à 30 % du poids total de la composition.

9. Composition selon l'une des revendications précédentes, se présentant sous forme d'émulsion ou de dispersion dans une phase huileuse, cireuse ou de solvant organique lipophile, de gel anhydre, pâte anhydre.

10. Utilisation cosmétique de la composition selon l'une des revendications précédentes, pour maquiller la peau et/ou les lèvres et/ou les phanères.

11. Utilisation de la composition selon l'une des revendications 1 à 9 pour la préparation d'un onguent destiné à traiter et/ou protéger la peau et/ou les lèvres et/ou les phanères.

12. Utilisation pour la fabrication d'une composition cosmétique colorée ou d'une composition dermatologique colorée, de vanadate de bismuth en vue de protéger la peau et/ou les lèvres et/ou les phanères contre les signes cutanés du vieillissement.

13. Procédé non thérapeutique de protection de la peau et/ou des lèvres et/ou des phanères, contre les signes cutanés du vieillissement photoinduit, consistant à appliquer sur la peau et/ou les lèvres et/ou les phanères la composition selon l'une des revendications 1 à 9.

14. Utilisation cosmétique dans une composition cosmétique colorée de vanadate de bismuth, comme agent pour limiter la production de radicaux libres ou protéger la peau et/ou les lèvres et/ou les phanères contre les radicaux libres.

15. Utilisation cosmétique de la composition selon l'une quelconque des revendications 1 à 9 pour traiter les signes cutanés du vieillissement.

## Claims

1. Coloured composition for topical application, containing a lipophilic continuous phase, **characterized in that** it comprises bismuth vanadate as colouring agent.

2. Composition according to Claim 1, **characterized in that** the colouring agent is present in a proportion of from 0.01 to 50% by weight relative to the weight of the composition.

3. Composition according to Claim 1 or 2, **characterized in that** the colouring agent is present in a proportion of from 0.5 to 25% by weight relative to the weight of the composition.

4. Composition according to one of the preceding claims, which is in the form of a make-up product for the skin, the lips and/or the superficial body growths of a human being.

5. Composition according to one of the preceding claims, which is in the form of a mascara, eyeliner, hair composition such as a lacquer, lip product, lip gloss, foundation, concealer product, face powder, eyeshadow, body make-up, powders or nail varnish.

6. Composition according to one of the preceding claims, **characterized in that** the fatty continuous phase contains at least one fatty substance chosen from oils, waxes, gums, pasty fatty substances, lipophilic organic solvents and mixtures thereof.

7. Composition according to one of the preceding claims, also comprising at least one aqueous, alcoholic or aqueous-alcoholic phase dispersed or emulsified in the lipophilic phase.

8. Composition according to one of the preceding claims, **characterized in that** it also contains an additional particulate phase which may be present in a proportion of from 0 to 30% of the total weight of the composition.

9. Composition according to one of the preceding claims, which is in the form of an emulsion or a dispersion in an oily or waxy phase or a lipophilic organic solvent, anhydrous gel or anhydrous paste.

10. Cosmetic use of the composition according to one of the preceding claims, to make up the skin and/or the lips and/or the superficial body growths.

11. Use of the composition according to one of Claims 1 to 9 for the preparation of an ointment intended to treat and/or protect the skin and/or the lips and/or the superficial body growths.

12. Use, for the manufacture of a coloured cosmetic composition or of a coloured dermatological composition, of bismuth vanadate in order to protect the skin and/or the lips and/or the superficial body growths against the cutaneous signs of ageing.

13. Non-therapeutic process for the protection of the skin and/or the lips and/or the superficial body growths against the signs of photo-induced ageing of the skin, which consists in applying the composition according to one of Claims 1 to 9 to the skin and/or the lips and/or the superficial body growths.

14. Cosmetic use, in a coloured cosmetic composition, of bismuth vanadate as an agent for limiting the production of free radicals or protecting the skin and/or the lips and/or the superficial body growths against free radicals.

15. Cosmetic use of the composition according to any one of claims 1 to 9, for treating the cutaneous signs of ageing.

## Patentansprüche

1. Farbige Zusammensetzung zur topischen Anwendung mit kontinuierlicher lipophiler Phase, **dadurch gekennzeichnet, daß** sie als Farbmittel Bismutvanadat enthält.

2. Zusammensetzung nach Anspruch 1, **dadurch gekennzeichnet, daß** das Farbmittel in einer Menge von 0,01 bis 50 Gew.-%, bezogen auf das Gewicht der Zusammensetzung, vorliegt.

3. Zusammensetzung nach Anspruch 1 oder 2, **dadurch gekennzeichnet, daß** das Farbmittel in einer Menge von 0,5 bis 25 Gew.-%, bezogen auf das Gewicht der Zusammensetzung, vorliegt.

4. Zusammensetzung nach einem der vorhergehenden Ansprüche, die als Produkt zum Schminken der menschlichen Haut, Lippen und/oder Hautanhangsgebilde vorliegt.

5. Zusammensetzung nach einem der vorhergehenden Ansprüche, die als Mascara, Eyeliner, Zusammensetzung für das Haar vom Typ Lack, Produkt für die Lippen, Lippenglanz, Make-up, Produkt gegen Augenringe, Wangenrouge, Lidschatten, Schminkprodukt für den Körper, Puder oder Nagellack vorliegt.

6. Zusammensetzung nach einem der vorhergehenden Ansprüche, **dadurch gekennzeichnet, daß** die kontinuierliche Fettphase mindestens eine Fettsubstanz enthält, die unter den Ölen, Wachsen, Gummis, pastösen Fettsubstanzen, lipophilen organischen Lösungsmitteln und deren Gemischen ausgewählt ist.

7. Zusammensetzung nach einem der vorhergehenden Ansprüche, die außerdem mindestens eine wäßrige, alkoholische oder wäßrig-alkoholische Phase enthält, die in der lipophilen Phase dispergiert oder emulgiert ist.

8. Zusammensetzung nach einem der vorhergehenden Ansprüche, **dadurch gekennzeichnet, daß** sie außerdem eine zusätzliche Partikelphase enthält, die in einer Menge von 0 bis 30 % des Gesamtgewichts der Zusammensetzung vorliegen kann.

9. Zusammensetzung nach einem der vorhergehenden Ansprüche, die als Emulsion oder Dispersion in einer Ölphase, Wachsphase oder einem lipophilen organischen Lösungsmittel, als wasserfreies Gel oder als wasserfreie Paste vorliegt.

10. Kosmetische Verwendung der Zusammensetzung nach einem der vorhergehenden Ansprüche zum Schinken der Haut und/oder der Lippen und/oder der Hautanhangsgebilde.

11. Verwendung einer Zusammensetzung nach einem der Ansprüche 1 bis 9 zur Herstellung einer Salbe, die zur Behandlung und/oder zum Schutz der Haut und/oder der Lippen und/oder der Hautanhangsgebilde vorgesehen ist.

12. Verwendung von Bismutvanadat zur Herstellung einer farbigen kosmetischen Zusammensetzung oder einer farbigen dermatologischen Zusammensetzung zum Schutz der Haut und/oder der Lippen und/oder der Hautanhangsgebilde gegen die Anzeichen der Hautalterung.

13. Nicht therapeutisches Verfahren zum Schutz der Haut und/oder der Lippen und/oder der Hautanhangsgebilde gegen die Anzeichen der lichtinduzierten Hautalterung, das darin besteht, auf die Haut und/oder die Lippen und/oder die Hautanhangsgebilde eine Zusammensetzung nach einem der Ansprüche 1 bis 9 aufzutragen.

14. Kosmetische Verwendung von Bismutvanadat in einer farbigen kosmetischen Zusammensetzung als Mittel, um die Bildung von freien Radikalen einzuschränken oder die Haut und/oder die Lippen und/oder die Hautanhangsgebilde gegen freie Radikale zu schützen.

15. Kosmetische Verwendung einer Zusammensetzung nach einem der Ansprüche 1 bis 9 zur Behandlung der Anzeichen von Hautalterung.
